# EUROPEAN PATENT SPECIFICATION

(11) **EP 1 005 478 B1**
(45) Date of publication and mention of the grant of the patent: **22.01.2003**
(21) Application number: 98948841.6
(22) Date of filing: 17.08.1998
(51) Int. Cl.: C07H 19/06, C07H 21/00, C07H 23/00, C07F 7/18

(54) **RIBONUCLEOSIDE-DERIVATIVE AND METHOD FOR PREPARING THE SAME**
RIBONUKLEOSID-DERIVAT UND VERFAHREN ZU SEINER HERSTELLUNG
DERIVE DE RIBONUCLEOSIDE ET SON PROCEDE DE PREPARATION

(30) Priority: 18.08.1997 CH 193197
(43) Date of publication of application: 07.06.2000
(73) Proprietor: Qiagen AG, 4052 Basel (CH)
(72) Inventor: Pitsch, Stefan, 8049 Zürich (CH); Weiss, Patrick A., 8051 Zürich (CH); Jenny, Luzi, 8004 Zürich (CH)
(74) Representative: Frei, Alexandra Sarah
(86) International application number: PCT/EP98/05215
(87) International publication number: WO 99/009044

(56) References cited:
- USMAN N ET AL: "AUTOMATED CHEMICAL SYNTHESIS OF LONG OLIGORIBONUCLEOTIDES USING 2'-O-SILYLATED RIBONUCLEOSIDE 3'-O-PHOSPHORAMIDITES ON A CONTROLLED-PORE GLASS SUPPORT: SYNTHESIS OF A 43-NUCLEOTIDE SEQUENCE SIMILAR TO THE 3'-HALF MOLECULE OF AN ESCHERICHIA COLI FORMYLMETHIONINE TRNA" JOURNAL OF THE AMERICAN CHEMICAL SOCIETY, vol. 109, no. 25, 9 December 1987, pages 7845-7854, XP000673490 cited in the application
- ANTONSEN, OEYVIND ET AL: "Preparation of monosilyl ethers of vicinal diols" 1992 , ACTA CHEM. SCAND. (1992), 46(8), 757-60 CODEN: ACHSE7;ISSN: 0904-213X XP002086772 See compound 11.
- GUNDERSEN, LISE LOTTE ET AL: "Aryl- and alkynyltriisopropoxytitanium reagents in regioselective carbon-carbon bond formation in azines" 1992 , TETRAHEDRON (1992), 48(27), 5647-56 CODEN: TETRAB;ISSN: 0040-4020 XP002086773 See examples 1 and 12 a in experimental.
- ANTONSEN, OEYVIND ET AL: "Cross aldol products from.alpha.-haloalkoxysilanes and silyl enol ethers" 1992 , ACTA CHEM. SCAND. (1992), 46(2), 172-7 CODEN: ACHSE7;ISSN: 0904-213X XP002086774 See compounds 4a-c.
- GUNDERSEN, LISE LOTTE ET AL: "Chloromethoxysilanes as protecting reagents for sterically hinered alcohols" 1989 , ACTA CHEM. SCAND. (1989), 43(7), 706-9 CODEN: ACHSE7 XP002086775 See compounds 4a-d.
- BENNECHE, TORE ET AL: "(tert-Butyldimethylsilyloxy)methyl chloride: synthesis and use as N-protecting group in pyrimidinones" 1988 , ACTA CHEM. SCAND., SER. B (1988), B42(6), 384-9 CODEN: ACBOCV;ISSN: 0302-4369 XP002086776 See structure 3.

## Description

### Field of the invention

The invention is in the field of nucleic acid chemistry and concerns a ribonucleoside-derivative and a method for preparing the ribonucleoside-derivative. The inventive ribonucleoside derivative is especially suitable for machine synthesis of ribonucleic acids.

### Background of the invention

The present invention is connected to the chemical synthesis of ribonucleic acids (ribo-oligonucleotides, RNA), especially to the machine synthesis of such oligomers as well as to the synthesis of structurally related derivatives of such oligomers.

Pure oligonucleotides of a defined sequence prepared in a chemical synthesis are e.g. used in the field of structural analysis of unit crystals by means of X-ray diffraction or by means of nuclear magnetic spectroscopy. This kind of research contributes to the understanding of biological processes on a molecular level and among other things make development of novel therapy concepts possible. Ribonucleic acids constituting a central biological class of compounds (messenger-RNA, transfer-RNA, ribosomal-RNA) are interesting objects for medical chemistry. In this context the availability of assays for fast and reliable testing of compounds potentially interacting with RNA is highly desireable. By chemical synthesis (opposed to production using enzymes, organisms etc.) of such oligonucleotides for testing, introduction of purposeful modifications becomes possible which modifications e.g. allow simple quantification of a desired interaction or make a specific interaction accessible to precise examination.

Natural and modified RNA-oligonucleotides also find use as tools for selective recognition and/or selective modification of RNA- and DNA-oligonucleotide-sequences and other compounds (aptamers and ribozymes). Improvements to be achieved in the chemical synthesis of such compounds could make the introduction of purposeful modifications possible and thus considerably extend the field of application of the types of compounds as described above in medical diagnostics and therapy.

All known methods for chemical synthesis of RNA-oligonucleotides and derivatives thereof are related to concepts which have been very successfully developed for the synthesis of DNA-oligonucleotides (2'-desoxyribonucleic acids, opposed to RNA which comprises a hydroxy-group in the 2'-position). The machine synthesis of DNA- and RNA-oligonucleotides is normally based on a protected nucleoside-derivative immobilized on a solid phase to which further protected nucleoside-derivatives are coupled in steps of one synthesis cycle each until the desired length of chain is achieved. Finally the built-up sequence is freed of all protection-groups and separated from the solid phase.

Ribonucleoside-derivatives for application in the chemical synthesis of ribonucleic acids comprise a D- or L-ribose-unit and have the following general structural formula: 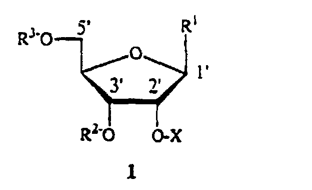 whereby
R¹ is a base of the purine- or pyrimidine-family or a derivative of such a base,
R² is a proton or a substituted derivative of phosphonic acid,
R³ is a proton or a protection-group for the oxygen atom in 5'-position,
X is a protection-group for the oxygen atom in 2'-position.

The protection-group X for the oxygen atom in 2'-position is to fulfil substantially the following conditions:
- The introduction of the protection-group has to be as simple as possible and has to yield uniform compounds which are as free from isomerization products as possible.
- The protection-group has to be absolutely stable under the coupling conditions.
- The protection-group has to have a structure which allows high coupling yields.
- The protection-group has to be completely removable without decomposition or chemical change of the compound to be prepared.

The protection-groups which have been used so far for the 2'-position substantially belong to the three following types. A number of further such protection-group types exist. However, they have not been completely successful for different reasons.
a) Acid-sensitive 2'-O-acetal-protection-groups [1]:
   Example: 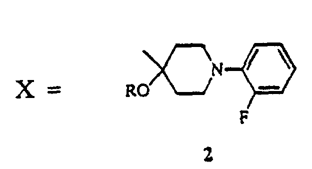
   This type of protection-group is easily introduced and the chemicals required for establishing such protection-groups as well as the nucleosides carrying them are commercially available. Disadvantages of the protection type are the facts that the protection-groups are not completely stable on synthesis of the chains, that using such protected nucleoside-derivatives only moderate coupling yields are achievable and that isomerization on de-protection is possible.
b) Photo-sensitive 2'-O-ortho-nitrobenzyloxymethyl-protection-groups [2]:
   Example: 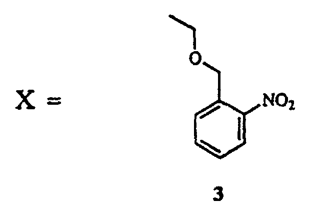
   This type of protection-group is easily introduceable, de-protecting is completely orthogonal and good coupling yields are achievable. Disadvantages of this type of protection-group are the facts that complete de-protecting is sometimes not possible and that the chemicals necessary for establishing the protection-groups are not available on the market.
c) Fluoride-sensitive 2'-O-silyl-protection-groups [3]:
   Example: 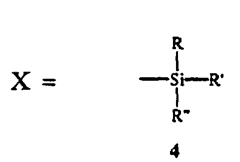
   This type of protection-group is the most successful one. It is well established in the industry since the early eighties and the necessary chemicals are available on the market. Protection groups of this type are easily and completely removable (de-protecting) and coupling yields of up to 98% can be achieved with coupling times in the order of ten to twenty minutes. This results in commercially reasonable yields in the order of 50% and more for oligonucleotides of up to about 35 units.

The yield of chain building corresponds to the yield of each coupling step to the power of the number of units contained in the chain. This means that increasing the coupling yield is not only advantageous for economic reasons but it makes it possible to build longer chains. Past and present efforts for increasing the coupling yield concentrate on the coupling parameters and resulted in the above mentioned maximum of 98%

The object of the invention is a substantial increase of the yield of RNA-chain building steps (coupling yield) without important changes to the chain building chemistry such that established equipment and established method steps are still applicable.

### Short description of the invention

This object is achieved by coupling ribonucleoside derivatives being protected in 2'-O-position with a novel protection group. As will be shown in the examples, using the novel ribonucleoside derivative coupling yields higher than 99% and possibly higher than 99.5% can be achieved. Furthermore, this coupling yield is achievable with a shorter coupling time, which constitutes a further advantage of the inventive ribonucleoside-derivative.

The coupling yield increase in the order of at least one percent as achieved by using the inventive ribonucleoside derivative results in a chain building yield in the order of 60 to 70% for a chain with 50 units (according to known methods: ca. 35%) and in the order of 40 to 60% for a chain of 100 units (according to known methods: ca. 13%).

The inventive nucleoside-derivatives unite the advantages of nucleoside-derivatives with known protection-groups as mentioned above under b) and c) (good introduceability of the protection-group, good stability of the protection-group, simple de-protecting) but do not show the known disadvantages in particular of the protection group as mentioned under c), which are isomerization and chain scission under de-protection conditions.

The inventive ribonucleoside-derivatives contain a 2'-O-silyloxymethyl-protection-group (structural formula 5), whereby the silicon atom of the silyloxymethyl-group additionally comprises three identical or different substituents. These three substituents are advantageously alkyl- or aryl-substituents. The three alkyl- or aryl-groups can also be aryl-alkyl-combinations, can be substituted with heteroatoms and/or can be connected to each other in ring-form. It shows that the three substituents of the 2'-O-silyloxymethyl-group can together comprise between 6 and 30 C-atoms.

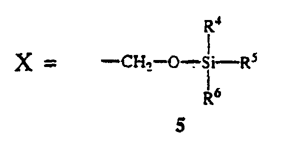

As an example the three substituents of the silicon atom in the 2'-O-silyloxymethyl-protection-group are three isopropyl-groups (structural formula 6)

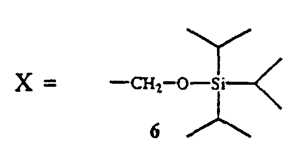

The advantages achieved by using the inventive ribonucleoside derivative for RNA-chain building may be explained as follows:
- Due to the acetal nature of the bond between nucleoside and protection-group in the inventive 2'-O-protected ribonucleoside-derivative no migration of the protection-group to a different position inside the ribonucleoside-derivative, in particular no migration to the neighboring 3'-O-position can occur. Such isomerization is an important and well known problem in the synthesis of the conventional 2'-O-silyl-substituted RNA-units (type of protection-group c), see above) [6, 7] which problem is solved for the inventive ribonucleoside-derivatives by the new protection-group.
- The 2'-O-silyloxymethyl-protection-group is less bulky because it is linked to the ribose unit via the relatively small and sterically undemanding methene-unit. This, in opposition to units with the known considerably more bulky trialkylsilyl-groups bonded directly to the 2'-oxygen atom (types of protection-groups c), see above) reduces steric hinderance of the reaction center for the coupling reaction (3'-O-phosphor atom) and thus increases the coupling yield.

As the difficulties of the chain formation caused by steric hindrance are greatly reduced by the methoxy-spacer of the protection-group of the inventive ribonucleic-acid-unit steric effects can be substantially neglected when choosing the three substituents of the silicon atom. Instead, additional criteria, in particular stability against acid and/or base can be taken into consideration. Furthermore, the protection-group can be adapted to a higher degree to the requirements of a specific case.

The separation of the 2'-O-silyloxymethyl-protection-group (de-protecting) can be carried out substantially under the same conditions as the separation of the conventional 2'-O-Silyl-protection-groups, i.e. by treatment with fluoride-ions or fluorosilicic acid. This de-protecting reaction is known to users of ribonucleic-acid-units as a well established and problem-free reaction and the fact that this reaction can be taken over for de-protecting the inventive units is a further advantage of these units.

For preparing the inventive compounds an efficient, cheap and simple method of synthesis is used, which method yields products substantially free of unwanted isomers (purity > 99.8%). By using the inventive ribonucleoside-derivatives, synthetically prepared oligo-nucleic-acids with chains (more than 40 nucleotide units) longer than previously possible become available and RNA-oligonucleotides in generally larger amounts (1 - 20 mg per individual synthesis) and in uniform, chemically pure form (over 90% in weight of the compound with the desired structure) become available for many interesting applications.

### Detailed description of the invention

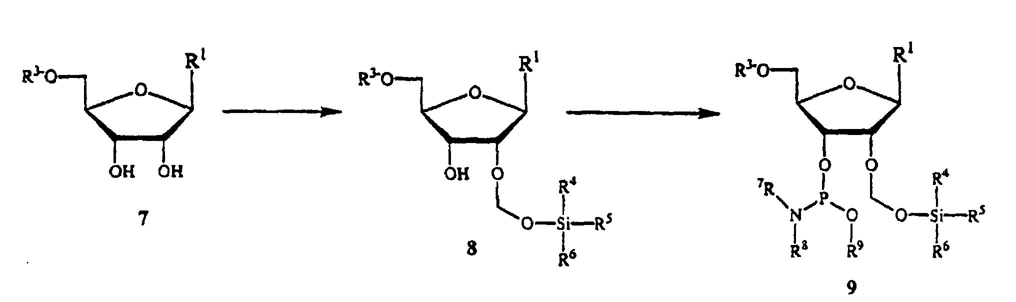

The reaction 7 → 8 → 9 shows an example of the synthesis of a compound according to the invention. This synthesis starts from nucleosides 7 which are already partly protected. A cyclic 2',3'-di-O-dialkyl-(or diaryl-)stannyl derivative (e.g. dibutyl-stannyl-derivative) is synthesized under alkaline conditions, in the presence of an excess of a tertiary amine base which derivative reacts with a tri(alkyl- and/or aryl)-substituted silyloxymethylchloride (or other halogenide) to form the ribonucleoside-derivative 8. This is then converted to the corresponding phosphoramidite 9 under established conditions [4].

As mentioned above the substituents R⁴, R⁵ and R⁶ of the 2'-O-silyloxymethyl-protection-group which protection-group distinguishes the inventive nucleoside-derivative, are identical or different alkyl- or aryl-substituents or combinations of these and can also be substituted with heteroatoms and/or be connected to each other forming ring structures. The three substituents together comprise advantageously between 6 and 30 carbon atoms. They are e.g. three isopropyl-groups.

The components and other substituents of the initial product which is not yet protected in 2'-O-position and of the protected product correspond precisely to the components and substituents which are used in conventional synthesis methods for preparing protected ribonucleoside-derivatives.

The protection-group R³ in 5'-O-position is e.g. a monomethoxytrityl- or dimethoxytrityl-group or a different, suitable group which is removed from the growing sequence during chain building such freeing a bonding position for coupling the next unit to be added to the chain.

The base-component R¹ of the ribonucleoside-derivative is a base of the purine- or pyrimidine family, e.g. one of the five nucleobases adenine, cytosine, thymine, uracile, guanine or a derivative thereof. It can be protected by an acyl-substituent which can be removed after chain creation.

The derivative of phosphonic acid in the 3'-O-position is an N,N- and O-substituted phosphoramidite group, whereby the N-substituents R⁷ and R⁸ are alkyl- or aryl-groups which can be further substituted and/or cyclically connected to each other. R⁷ and R⁸ are e.g. isopropyl-groups. By activating the nitrogen of the disubstituted amino-group the phosphorus center is activated for coupling the unit to a growing chain.

The 0-substituent R⁹ of the phosphoramidite-group is an alkyl- or aryl-substituent (possibly substituted by heteroatoms) which is removed after chain creation.

One skilled in the art of oligonucleotide-synthesis knows the principles of the synthesis of the inventive ribonucleoside-derivatives and their coupling to form oligo-nucleotides. For further illustration of the simplicity of the synthesis and the superiority of the inventive units for the synthesis of ribonucleic acids several examples follow.

### Example 1

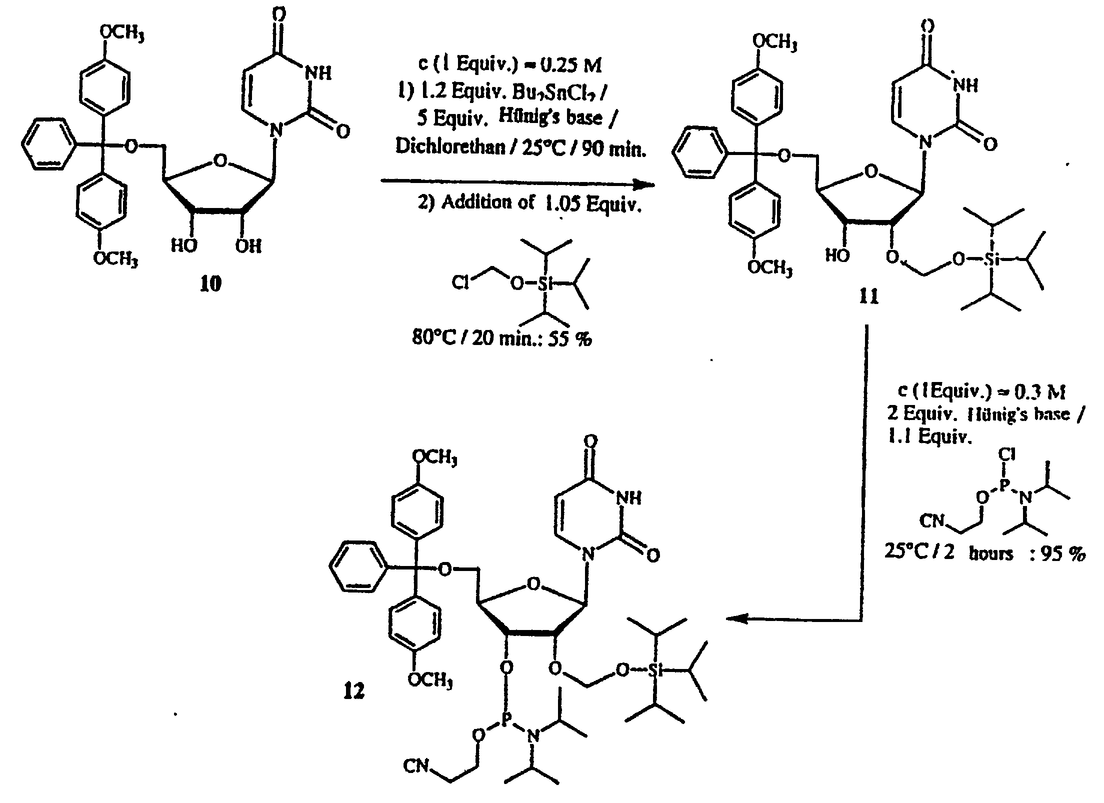

The synthesis of the uridine-unit 1-{3'-O-[(2-cyanoethoxy)(diisopropylamino)-phosphino]-5'-O-[4,4'-dimethoxytrityl]-2'-O-[triisopropyl-silyloxymethyl]-β-D-ribofuranosyl}-uracile 12 was carried out starting from 1-[5'-O-(4,4'-dimethoxytrityl)-β-D-ribofuranosyl]-uridine 10 and carried out via the intermediate product 11: 1-[5'-O-(4,4'-dimethoxytrityl)-2'-O-(triisopropylsilyloxymethyl)-β-D-ribofuranosyl]-uracile.

The protected ribonucleoside-derivative 11 was purified to an isomerically pure form by means of simple chromatography on silica gel.

### Example 2

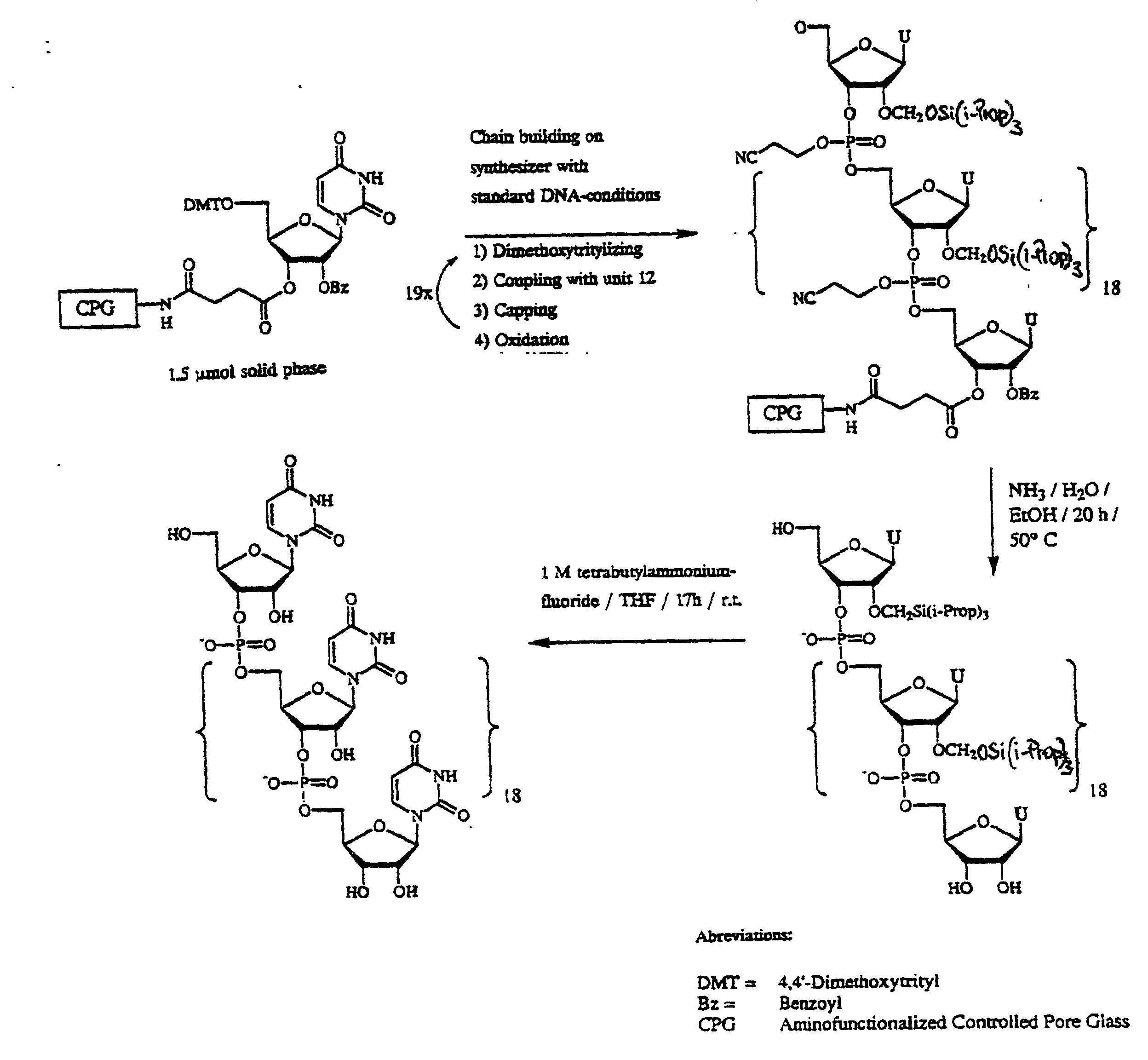

With a coupling time of 2 minutes, coupling yields as indicated by the detritylation assay built into commercially available chain building equipment were found to be as follows:

| electrophile unit | nucleophile unit | coupling yield |
|---|---|---|
| adenine | adenine | 99.6% |
| adenine | cytosine | 99.0% |
| adenine | guanine | 99.1% |
| adenine | uracile | 99.5% |
| cytosine | adenine | 99.1% |
| cytosine | cytosine | 99.3% |
| cytosine | guanine | 98.4% |
| cytosine | uracile | 99.5% |
| guanine | adenine | 99.5% |
| guanine | cytosine | 99.5% |
| guanine | guanine | 99.9% |
| guanine | uracile | 99.9% |
| uracile | adenine | 99.5% |
| uracile | cytosine | 99.1% |
| uracile | guanine | 98.4% |
| uracile | uracile | 99.9% |

This results in a mean coupling yield of 99.3%

### Example 3: Procedure for the preparation of (Chloromethoxy)(triisopropyl)-silane (according to [8])

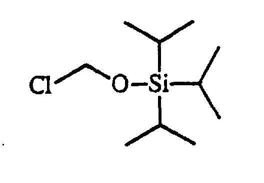

A suspension of 0.1 mol para-formaldehyde in 0.1 mol ethanethiol was treated with 1 drop 10N aequous NaOH-solution and stirred at room temperature until a clear solution was obtained. After stirring for 1 hour at 50°C, 50 ml CH₂Cl₂ and 0.2 mol imidazole, followed by 0.09 mol (i-Prop)₃SiCl were added. The resulting suspension was stirred at room temperature overnight and diluted with 400 ml hexane. After addition of 250 ml aequous 2M NaH₂PO₄- solution, stirring and phase separation, the organic phase was evaporated. The residue was dissolved in 250 ml CH₂Cl₂, treated with 0.09 mol sulfurylchloride, stirred 1 hour at room temperature, evaporated and distilled in vacuo. The product was obtained as colourless, viscous oil (yield: 90%).

Boiling point: 50°C (0.1 torr). ¹H-NMR (300 MHz, CDCl₃): 1.08 - 1.10 (*m*, 21 H, CH₃ and CH from (i-Prop)₃Si-); 5.66 (*s*, 2 H, CH₂Cl).

For preparing silyloxymethylchloride with other substituents than isoporpyl as well as for preparing other silyloxymethylhalogenides, the procedure as given above is adapted correspondingly.

### Example 4: Procedure for the preparation of the 2'-O-[(i-Prop)₃SiOCH₂]-protected nucleosides 5 - 8

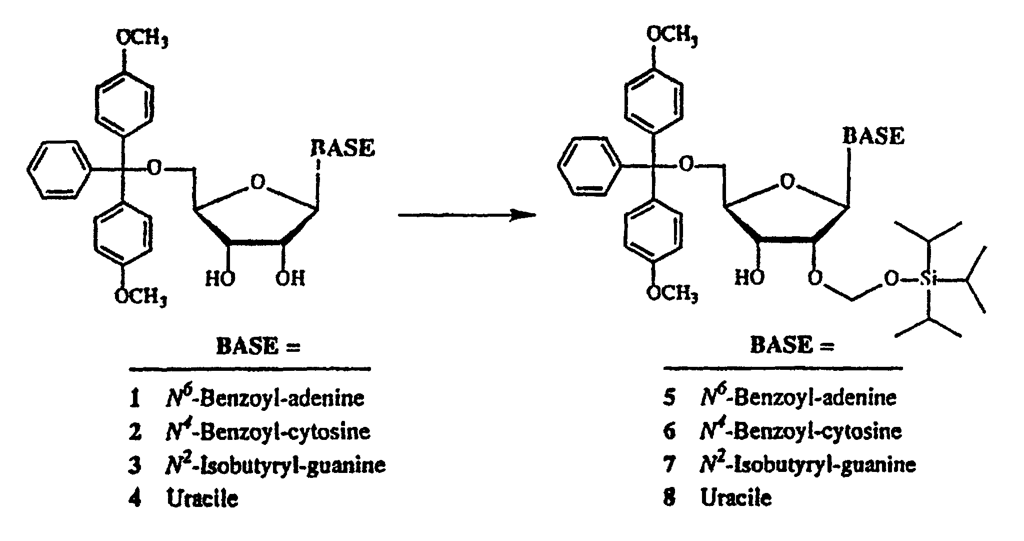

A solution of 10 mmol 5'-O-dimethoxytritylated, eventually base protected nucleoside 1 - 4 (preparation according to [9]) in 40 ml 1,2-dichloroethane was treated at room temperature first with 50 mmol N-ethyl-N,N-diisopropylamine and then with 11 mmol dibutyltin dichloride. After stirring for 15 minutes at room temperature, the reaction mixture was heated to 80°C, treated with 13 mmol (chloromethoxy)(triisopropyl)-silane and stirred for 30 to 90 minutes at 80°C, until only traces of starting material could be detected by thin-layer-chromatography. After cooling to room temperature, the reaction mixture was diluted with 200 ml CH₂Cl₂, 200 ml aqueous saturated NaHCO₃-solution were added and the resulting mixture was stirred for 20 minutes. The cloudy organic phase obtained after phase separation was dried over MgSO₄ and filtered through a pad of Celite(Trade mark). The residue, obtained after concentration, was subjected to column-chromatography on 100 g of silica gel using as eluent hexane/ethyl acetate mixtures, containing 2 % NEt₃. The products were obtained as colorless foams.

### N⁶-Benzoyl-9-[5'-O-(4,4'-dimethoxytrityl)-2'-O-([(triisopropylsilyl)oxy]methyl))-β-D-ribofuranosyl]adenine (5):

Yield: 45 - 55 %.
TLC: *R*_{f} 0.60 (AcOEt / hexane 7:3). ¹H-NMR (300 MHz, CDCl₃): 1.03 - 1.15 (*m,* 21 H, CH₃ and CH from (i-Prop)₃Si-); 3.08 (*d*, *J* = 3.7, 1 H, HO-C(3'), disappears upon treatment with D₂O); 3.40 *(d* x *d, J* = 10.2, 4.1, 1 H, H-C(5')); 3.62 (*d* x *d, J =* 10.2, 3.5 Hz, 1 H, H'-C(5')); 3.78 (*s*, 6 H, CH₃O-Ar); 4.31 (*q*, *J* = 4.0, 1 H, H-C(4')); 4.57 (br. *q*, *J* Å 4, 1 H, H-C(3'), changes to *t* upon treatment with D₂O); 4.98 (br. *t*, *J* Å 5, 1 H, H-C(2')); 4.98 and 5.16 (two *d, J =* 4.7, 2 H, OC*H*₂O); 6.24 *(d, J =* 5.6, 1 H, H-C(1')); 6.79 - 6.83 *(m,* 4 H, arom. H); 7.21 - 7.65 *(m,* 12 H, arom. H); 8.01 - 8.04 (*m*, 2 H, arom. H); 8.21 (*s*, 1 H, H-C(2)); 8.73 (*s*, H-C(8)); 8.97 (br. *s*, 1 H, N*H*-C(6), disappears upon treatment with D₂O).

### N⁴-Benzoyl-1-[5'-O-(4,4'-dimethoxytrityl)-2'-O-([(triisopropylsilyl)oxy]methyl))-β-D-ribofuranosyl]cytosine (6):

Yield: 50 - 60 %.
TLC: *R*_{f} *0.65* (AcOEt / hexane 7:3). ¹H-NMR (300 MHz, CDCl₃): 1.02 - 1.18 (*m*, 21 H, CH₃ and CH from (i-Prop)₃Si-); 3.34 (*d*, *J* = 8.3, 1 H, HO-C(3'), disappears upon treatment with D₂O); 3.55 *(d* x *d, J =* 11.6, 3.0, 1 H, H-C(5')); 3.62 *(d* x *d, J* = 11.6, 3.0 Hz, 1 H, H'-C(5')); 3.83 (*s*, 6 H, CH₃O-Ar); 4.12 *(d* x *t*, *J* = 8.3, 3.0, 1 H, H-C(4')); 4.28 (*d*, *J =* 5.4, 1 H, H-C(2')); 4.41 (*t* x *d*, *J =* 8.3, 5.4, 1 H, H-C(3'), changes to *d* x *d* upon treatment with D₂O); 5.19 and 5.30 (two *d, J = 4.6,* 2 H, OC*H*₂O); 6.01 (*s*, 1 H, H-C(1')); 6.84 - 6.92 (*m*, 4 H, arom. H); 7.23 - 7.62 (*m*, 13 H, 12 arom. H and H-C(5)); 7.85 - 7.92 *(m,* 2 H, arom. H); 8.54 (*d*, *J = 6.5,* 1 H, H-C(6)); 8.55, (br. *s,* 1 H, *H*N-C(4), disappears upon treatment with D₂O).

### N³-Isobutyryl-9-[5'-O-(4,4'-dimethoxytrityl)-2'-O-([(triisopropylsilyl)oxy]methyl))-β-D-ribofuranosyl]guanine (7):

Yield: 80 - 90 %.
TLC: *R*_{f} 0.50 (AcOEt / hexane 7:3). ¹H-NMR (300 MHz, CDCl₃): 0.66, 0.87 (2*d*, *J* = 6.9, 6 H, CH(C*H*₃)₂); 1.02 - 1.11 *(m,* 21 H, CH₃ and CH from (i-Prop)₃Si-); 1.49 (*hept*, *J* = 6.9, 1 H, C*H*(CH₃)₂); 3.02 (*d,* J = 1.9. 1 H, HO-C(3'), disappears upon treatment with D₂O); 3.00 *(dd, J =* 3.1, 10.6, 1 H, H-C(5')); 3.54 (*dd*, *J =* 2.1, 10.6, 1 H, H'-C(5')); 3.76, 3.77 (2*s*, 2 x 3 H, OC*H*₃); 4.22 (br. *q*, *J* Å 2, 1 H, H-C(4')); 4.57 *(m,* 1 H, H-C(3'), changes to *d* x *d* upon treatment with D₂O); 4.95, 5.14 (2*d*, *J* = 4.7, 2 H, OC*H*₂O); 5.08 (*dd*, *J* = 5.1, 7.2, 1 H, H-C(2')); 5.89 (*d*, *J =* 7.2, 1 H, H-C(1')); 6.77-6.82 (*m*, 4 H, arom. H); 7.21-7.57 *(m,* 9 H, arom. H); 7.77 (br. *s*, N*H-*C(2)); 7.79 (*s*, 1 H, H-C(8)); 11.95 (br. *s*, 1 H, H-N(1), disappears upon treatment with D₂O).

### 1-[5'-O-(4,4'-dimethoxytrityl)-2'-O-([(triisopropylsilyl)oxy]methyl))-β-D-ribofuranosyl]uracile (8):

Yield: 45 - 55 %.
TLC: *R*_{f} 0.75 (AcOEt / hexane 3:2). ¹H-NMR (300 MHz, CDCl₃): 1.02 - 1.18 *(m,* 21 H, CH₃ and CH from (i-Prop)₃Si-); 3.17 *(d, J* = 5.5, 1 H, HO-C(3'), disappears upon treatment with D₂O); 3.51 *(d, J* = 2.5, 2 H, H-C(5') and H'-C(5')); 3.80 (*s*, 6 H, CH₃O-Ar); 4.12 *(d* x *t*, *J =* 5.5, 2.5, 1 H, H-C(4')); 4.28 *(d* x *d, J* = 3.2, 5.5, 1 H, H-C(2')); 4.47 (*q*, *J* = 5.5, 1 H, H-C(3'), changes to *t* upon treatment with D₂O); 5.04 and 5.23 (two *d*, *J* = 5.0, 2 H, OC*H*₂O); 5.30 (*d*, *J =* 7.9, 1 H, H-C(5)); 6.03 *(d,* J = 3.2, 1 H, H-C(1')); 6.80 - 6.88 (*m*, 4 H, arom. H); 7.24 - 7.42 (*m*, 9 H, arom. H); 7.94 (*d*, *J* = 7.9, 1 H, H-C(6)); 8.56, (br. *s*, 1 H, H-N(3), disappears upon treatment with D₂O).

### Example 5: Procedure for the preparation of the Phosphoramidites 9 - 12

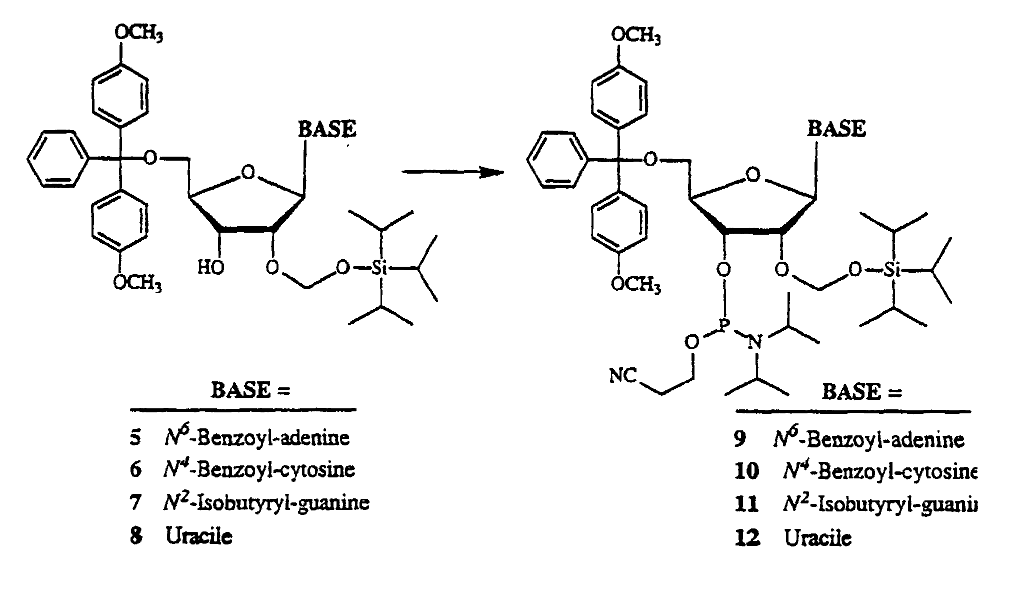

A solution of 10 mmol protected nucleoside 5 - 8 in 30 ml CH₂Cl₂ was treated consecutively with 20 mmol N-ethyl-N,N-diisopropylamine and 20 mmol chloro(2-cyanoethoxy)(N,N-diisopropylamino)phosphine [4]. After stirring for 3 h at room temperature, the reaction mixture was subjected to column-chromatography on 150 g of silica gel using as eluent hexane / ethyl acetate mixtures, containing 2 % NEt₃. The products were obtained as colorless foams (mixture of diastereoisomers).

### N⁶-Benzoyl-9-[5'-O-(4,4'-dimethoxytrityl)-2'-O-([(triisopropylsilyl)oxy]methyl))-β-D-ribofuranosyl]adenine 3'-[(2-Cyanoethyl) Diisopropylphosphoramidite] (9):

Yield: 90 - 95 %.
TLC: *R*_{f} 0.30 (hexane / EtOAc 7:3). ¹H-NMR (300 MHz, CDCl₃): 0.89 - 1.22 *(m,* 33 H, CH₃ from (i-Prop)₂N-; CH₃ and CH from (i-Prop)₃Si-); 2.39 (*t*, *J* = 6.5, 1 H, CH₂CN); 2.65 (*dt*, *J* = 1.2, 6.2, 1 H, CH₂CN); 3.36 (*m*, 1 H, OCH₂); 3.51 - 3.73 (*m*, 4 H, OCH₂, CH from (i-Prop)₂N-, H-C(5')); 3.77, 3.78 (2*s*, 6 H, OCH₃); 3.84 - 3.99 *(m,* 1 H, H'-C(5')); 4.37, 4.42 (2*m*, 1 H, H-C(4')); 4.65 *(m,* 1 H, H-C(3')); 4.94 - 5.02 *(m,* 2 H, OCH₂O); 5.24 *(m,* 1 H, H-C(2')); 6.20, 6.23 (*2d*, *J* = 5.6, 1 H, H-C(1')); 6.75-6.81 *(m,* 4 H, arom. H); 7.21-7.61 *(m,* 12 H, arom. H); 7.99-8.04 (*m*, 2 H, arom. H); 8.18, 8.20 (2*s*, 1 H, H-C(2)); 8.69, 8.72 (2*s*, 1 H, H-C(8)); 9.01 (br. *s*, 1 H, NH-C(6)). ³¹P-NMR (120 MHz, CDCl₃): 150.8, 151.6.

### N⁴-Benzoyl-9-(5'-O-(4,4'-dimethoxytrityl)-2'-O-([(triisopropylsilyl)oxy]methyl))-β-D-ribofuranosyl]cytosine 3'-[(2-Cyanoethyl) Diisopropylphosphoramidite] (10):

Yield: 90 - 95 %.
TLC: *R*_{f} 0.50/0.45 (hexane / EtOAc 7:3). ¹H-NMR (300 MHz, CDCl₃): 0.99 - 1.23 (*m*, 33 H, CH₃ from (i-Prop)₂N-; CH₃ and CH from (i-Prop)₃Si-); 2.39 (*t*, *J* = 6.3, 1 H, CH₂CN); 2.61 (*dt*, *J =* 2.5, 6.2, 1 H, CH₂CN); 3.43 - 3.97 *(m,* 12 H, OCH₂, CH from (i-Prop)₂N-, H and H'-C(5'), OCH₃); 4.29 - 4.56 (*m*, 3 H, H-C(2',3',4')); 5.20 (*s*, 2 H, OCH₂O); 6.18, 6.19 (2*d*, *J* = 2.0, 1 H, H-C(1')); 6.84-6.89 *(m,* 4 H, arom. H); 7.26-7.63 (*m*, 13 H, arom. H, H-C(5)); 7.88 *(m,* 2 H, arom. H); 8.41, 8.51 (2 *d, J* = 7.5, 1 H, H-C(6)); 8.40 (br. *s*, 1 H, NH-C(4)). ³¹P-NMR (120 MHz, CDCl₃): 150.7, 150.9.

### N²-Benzoyl-9-[5'-O-(4,4'-dimethoxytrityl)-2'-O-([(triisopropylsilyl)oxy]methyl))-β-D-ribofuranosyl]guanine 3'-[(2-Cyanoethyl) Diisopropylphosphoramidite] (11):

Yield: 90 - 95 %.
TLC: *R*_{f} 0.55 (hexane / EtOAc 1:1). ¹H-NMR (300 MHz, CDCl₃): 0.75 - 1.29 (*m*, 39 H, CH₃ from (i-Prop)₂N- and (i-Prop)₂CHCOO-; CH₃ and CH from (i-Prop)₃Si-); 1.62, 1.91 (2*hept*, *J* = 6.9, 1 H, CH from (i-Prop)₂CHCOO-); 2.26, (*t*, *J* = 6.6, 1 H, CH₂CN); 2.74 (*dt*, *J* = 1.1, 6.8, 1 H; C*H*₂CN); 3.19 (*m*, 1 H, OCH₂); 3.45-3.69 (*m*, 3 H, CH from (i-Prop)₂N-, OCH₂); 3.756, 3.761, 3.765 (3*s*, 6 H, OCH₃); 3.88 - 4.17 *(m,* 2 H, H,H'-C(5')); 4.22, 4.32 (2br. *s*, 1 H, H-C(4')); 4.58 *(m,* 1 H, H-C(3')); 4.89 - 4.98 *(m,* 2 H, OCH₂O); 5.07, 5.16 (2*dd*, *J* = 4.7, 7.6, 1 H, H-C(2')); 5.84, 5.96 (2*d, J* = 7.6, 1 H, H-C(1')); 6.76 - 6.81 *(m,* 4 H, arom. H); 7.21 - 7.55 (*m*, 9 H, arom. H); 7.74, 7.79 (2*s*, 1 H, H-C(8)); 7.87, 8.26 (2br. *s*, 1 H, NH-C(2)); 11.97 (br. *s*, 1 H, H-N(1)). ³¹P-NMR (120 MHz, CDCl₃): 150.4, 150.7.

### 1-[5'-O-(4,4'-dimethoxytrityl)-2'-O-([(triisopropylsilyl)oxy]ethyl))-β-D-ribofuranosyl]uracile 3'-[(2-Cyanoethyl) Diisopropylphosphoramidite] (12):

Yield: 90 - 95 %.
TLC: *R*_{f}0.50 (hexane / EtOAc 7:3). ¹H-NMR (300 MHz, CDCl₃): 1.02 - 1.18 *(m, 33* H, CH₃ from (i-Prop)₂N-; CH₃ and CH from (i-Prop)₃Si-); 2.39 (*t*, *J* = 6.6, 1 H, CH₂CN); 2.53 (*m*, 1 H, OCH₂); 2.64 (*dt*, *J =* 1.5, 6.2, 1 H, CH₂CN); 3.39 *(m,* 1 H, OCH₂); 3.52 - 3.69 (*m*, 3.5 H, CH from (i-Prop)₂N-, H,H'-C(5')); 3.78, 3.79, 3.80 (3*s*, 6 H, OCH₃); 3.82 - 3.96 (*m*, 0.5 H, H'-C(5')); 4.19, 4.27 (2br. *s*, 1 H, H-C(4')); 4.39 - 4.49 (*m*, 2 H, H-C(2',3')); 4.98 - 5.07 (*m*, 2 H, OCH₂O); 5.32, 5.36 (2*d*, *J* = 8.1, 1 H, H-C(5)); 6.12 *(d, J* = 4.4, 0.5 H, H-C(1')); 6.13 *(d, J* = 4.2, 0.5 H, H-C(1')); 6.81-6.86 *(m,* 4 H, arom. H); 7.23-7.43 *(m,* 9 H, arom. H); 7.81, 7.86 (2*d*, *J* = 8.1, 1 H, H-C(6)); 8.75 (br. *s*, 1 H, H-N(2)). ³¹P-NMR (120 MHz, CDCl₃): 150.9, 151.3.

### Example 6: Procedure for the synthesis of ribonucleic acids with Phosphoramidites 9 - 12

In a typical synthesis, commercially available "Controlled Pore Glass" supports loaded with 2 µmoles of the appropriately protected ribonucleosides (from *Sigma)* were used on a DNA-synthesizer (*Pharmacia* Gene Assembler). The original protocol of the manufacturer [10], developed for the synthesis of DNA-oligonucleotides in a 1.3 µmol scale, was used with the following exceptions: For each coupling 0.16 ml of a 0.08 M (= 1.28 µmoles) phosphoramidite solution was employed and the coupling time was adjusted to 12 minutes. Coupling yields determined by the built-in detritylation assay were on average above 99% with individual coupling yields of up to 99.9%.

HPLC Traces of crude oligoribonucleotides obtained from posphoramidites 9 to 12 are shown in the following Figures 1 and 2. The parameters used for the preparation were as follows:
1 10 M CH₃NH₂ in H₂O / EtOH 1 : 1; 25°C, 5 hours
2 1 M Tetrabutylammonium fluoride in THF; 25°C, 5 hours
3 chromatography on reversed phase columns

The sequence r(UUUUUUUUUUUUUUUUUUUU) was produced with an overall coupling yield of 87%, the sequence r(GCUCGUCUGAUGAGUCCGUGAGGACGAAAGACCGU) with an overall coupling yield of 74%.

### Example 7

The following chains were synthesized with the method as indicated in Example 2 and with the following mean coupling yields as indicated by the detritylation assay.

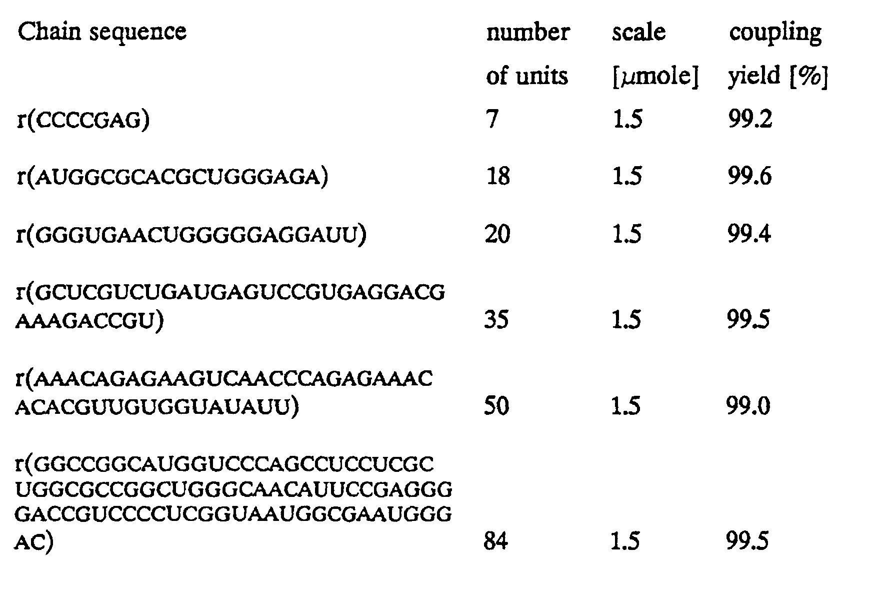

### Example 8

For a comparison between RNA-chain building using the inventive ribonucleoside derivatives and RNA-chain building using the known ribonucleoside derivatives with a silyl-protection group on the oxygen atom in 2'-position, various chains were synthesized using either phosphoramidites A or B:

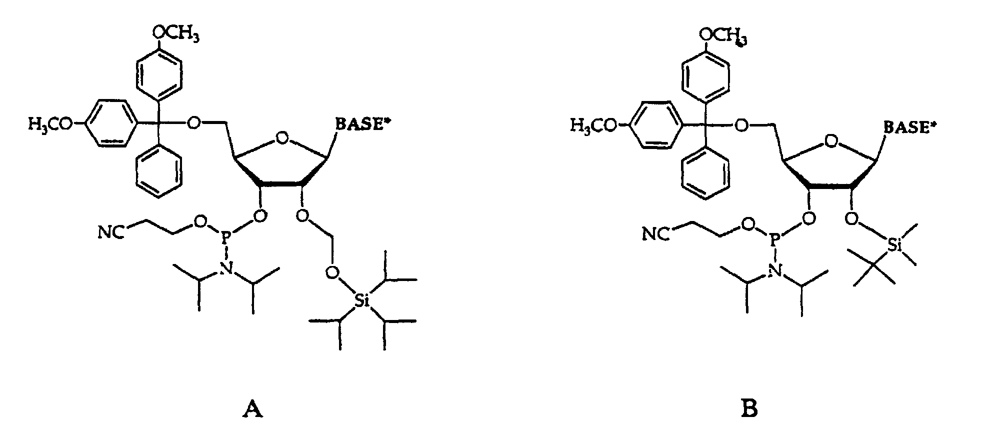

For RNA-synthesis using 1.5 µmole of solid support, 120 µl (0.1M) of phosphoramidite solution (mole-equivalents: 8) and 360 µl of 0.25M-benzylthiotetrazol the following mean coupling yields (according to detritylation assay) were found:

| Phosphoramidite | coupling time | mean coupling yield |
|---|---|---|
| A | 12 min. | >99.5% |
| A | 15 min. | >995% |
| B | 12 min. | max. 98% |
| B | 1.5 min | ca. 92% |

The results show clearly the different dependence on the coupling time between the synthesis using the two phosphoramidites.

For base de-protection (ammonolysis), the chains built from phosphoramidites A were treated for 2 hours with 8M methylamine in ethanol/water (1:1). No degradation was found with ammonolysis up to 24 hours. The chains built from the phosphoramidites B were treated according to the state of the art during 30 min. with 4M methylamine and 7M ammonia in water.

For de-protection of the 2'O-position, the chains built from phosphoramidites A were treated during 3 hours and the chains built from phosphoramidites B during 14 to 26 hours with tetrabutylammonium fluoride in water. Further treatment of the chains built from phosphoramidites A for up to 48 hours did not result in any chain scission.

### Literature

[1] B.E. Griffin. M. Jarman, C.B. Reese, Tetrahedron 1968, 24, 639; D.G. Norman, C.B. Reese, H.T. Serafinowska, Tetrahedron Lett. 1984, 25, 3015; C.B. Reese, R. Saffhill, J.E. Sulston, Tetrahedron 1970, 26, 1023; M.V. Rao, C.B. Reese, V. Schehlman, P.-S. Y. Chen, Chem. Soc. Perk. Trans. 1, 1993, 43; D.C. Capaldi, C.B. Reese, Nucleic Acids Res. 1994, 22, 2209; M.V. Rao, P. Macfarlane, Nucleos. Nucleot. 1995, 14, 911.
[2] M.E. Schwartz, R.R. Breaker, G.T. Asteriadis, J.S. deBear, G.R. Gough, Bioorg. Med. Chem. Lett. 1992, 2, 1019; S. Pitsch, Helv. Chim. Acta 1997, in preparation.
[3] K.K. Ogilvie, K.L. Sadana, E.A. Thompson, M.A. Quilliam, J.B. Westmore, Tetrahedron Lett. 1974, 15, 2861; K.K. Ogilvie, S.L. Beaucage, A.L. Schifman, N.Y. Theriault, K.L. Sadana, Can. J. Chem. 1978, *56,* 2768; D. Flockerzi, G. Silber, R. Charubala, W. Schlosser, R.S. Varma, F. Creegan, Liebigs Ann. Chem. 1981, 1568; N. Usman, K.K. Ogilvie, M.-Y. Jiang, R.J. Cedergren, J. Am. Chem. Soc. 1987, 109, 7845; K.K. Ogilvie, N. Usman, K. Nicoghosian, RJ. Cedergren, Proc. Natl. Acad. Sci. U.S.A. 1988, 85, 5764.
[4] N.D. Sinha, J. Biernat, H. Köster, Tetrahedron Lett. 1983, 24, 5843; N.D. Sinha, J. Biernat, J. McManus, H. Koster, Nucleic Acids Res. 1984, 12, 4539.
[5] M.D. Matteucci, M.H. Caruthers, Tetrahedron Lett. 1980, 21, 3243; M.D. Matteucci, M.H. Caruthers, J. Am. Chem. Soc. 1981, 3185; S.L. Beaucage, M.H. Caruthers M.H., Tetrahedron Lett. 1981, 22, 1859; L.J. McBride, M.H. Caruthers, ibid. 1983, 24, 245; R.L. Letsinger, K.K. Ogilvie, J. Am. Chem. Soc. 1969, 91, 3350.
[6] S.J. Jones and C.B. Reese, J. Chem. Soc. Perkin I, 1979 2762.
[7] N. Usman, K.K. Ofilvie, M.Y. Jiang amd R.L. Cedergren, J. Am. Chem. Soc., 109, 7845.
[8] L.-L. Gundersen, T. Benneche, K. Undheim, Acta Chem. Scand. 1989, 43, 706.
[9] G. H. Hakimelahi, Z. A. Proba, K. K. Ogilvie, Can. J. Chem. 1982, 60, 1106.
[10] Pharmacia, 'User Manual for Gene Assembler Plus'.

## Claims

1. Ribonucleoside-derivative comprising a D- or L-ribose-unit and having the structural formula 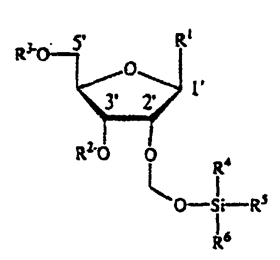 wherein
R¹ is a base of the purine- or pyrimidine-family or a derivative of such a base,
R² is a proton or a substituted derivative of phosphonic acid,
R³ is a proton or a suitable protection-group,
and R⁴, R⁵ and R⁶ are three aryl- or alkyl- substituents together comprising six to thirty C-atoms.

2. Ribonucleoside-derivative according to claim 1, **characterized in that** the substituents (R⁴, R⁵, R⁶) on the silicon of the silyloxymethyl-group in 2'-O-position are at least partly aryl-substituted alkyl-groups or alkyl-substituted aryl-groups.

3. Ribonucleoside-derivative according to claim 1, **characterized in that** the substituents (R⁴, R⁵, R⁶) on the silicon of the silyloxymethyl-group in 2'-O-position are at least partly substituted with heteroatoms.

4. Ribonucleoside-derivative according to claim 1, **characterized in that** the substituents (R⁴, R⁵, R⁶) on the silicon of the silyloxymethyl-group in 2'-O-position are at least partly interconnected.

5. Ribonucleoside-derivative according to claim 1, **characterized in that** the substituents (R⁴, R⁵, R⁶) on the silicon of the silyloxymethyl-group in 2'-O-position are isopropyl-groups.

6. Ribonucleoside-derivative according to claim 1, **characterized in that** the base (R¹) is cytosine, guanine, adenine, uracile or thymine.

7. Ribonucleoside-derivative according to claim 1, **characterized in that** the base (R¹) carries an acyl-protection-group.

8. Ribonucleoside-derivative according to claim 1, **characterized in that** the substituted derivative of phosphonic acid (R²) is an O- and N,N-substituted amino-phosphino-group.

9. Ribonucleoside-derivative according to claim 8, **characterized in that** the O-substituent is a 2-cyanoethyl-group and that the N-substituents are isopropyl groups.

10. Ribonucleoside-derivative according to claim 1, **characterized in that** the protection group (R³) of the oxygen in 5'-position is a monomethoxytrityl- or a dimethoxytrityl-group.

11. Use of the ribonucleoside-derivative according to claim 1 for the chemical synthesis of RNA-oligonucleotides with a predetermined nucleotide-sequence.

12. Method for the preparation of the ribonucleoside-derivative according to claim 1, **characterized in that** a cyclic 2',3'-di-O-stannyl-derivative is prepared in the presence of a base from a ribonucleoside the oxygen in 5'-position of which is protected by a protection-group and that this stannyl-derivative is converted to the ribonucleoside-derivative by addition of a silyloxymethylhalogenide carrying three additional substituents on the silicon atom.

13. Method according to claim 12, **characterized in that** in a further step the ribonucleoside-derivative is substituted on the oxygen in 3'-position with a group comprising a derivative of phosphonic acid.

14. A silyloxymethylhalogenide of the formula 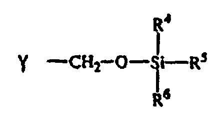 wherein Y is halogen and R⁴, R⁵ and R⁶ are isopropyl.

15. A silyloxymethylhalogenide of the formula 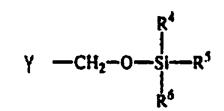 wherein Y is halogen and R⁴, R⁵ and R⁶ are alky- or aryl substituents together comprising between six and thirty C-atoms and being at least partly interconnected.

## Patentansprüche

1. Ribonukleosid-Derivat, welches eine D- oder L-Riboseeinheit umfasst und die folgende Strukturformel hat 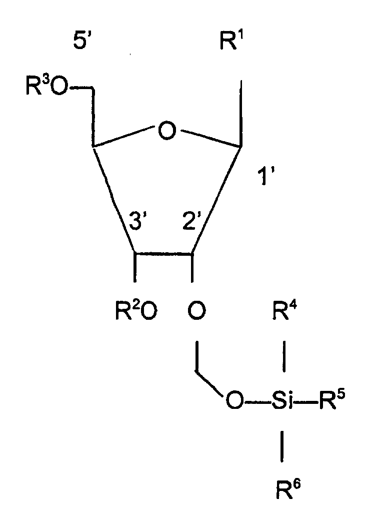 wobei
R¹ eine Base aus der Purin- oder Pyrimidin-Familie oder ein Derivat einer solchen Base ist,
R² ein Proton oder ein substituiertes Derivat einer phosphonischen Säure ist,
R³ ein Proton oder ein geeignete Schutzgruppe ist,
und R⁴, R⁵ und R⁶ drei Aryl- oder Alkylsubstituenten sind, welche zusammen 6 bis 30 C-Atome aufweisen.

2. Ribonukleosid-Derivat nach Anspruch 1, **dadurch gekennzeichnet, dass** die Substituenten an dem Silizium der Silyloxymethyl-Gruppe in 2'-O-Position zumindest teilweise arylsubstituierte Alkylgruppen oder alkylsubstituierte Arylgruppen sind.

3. Ribonukleosid-Derivat nach Anspruch 1, **dadurch gekennzeichnet, dass** die Substituenten (R⁴, R⁵, R⁶) an dem Silizium der Silyloxymethyl-Gruppe in 2'-O-Position zumindest teilweise mit Heteroatomen substituiert sind.

4. Ribonukleosid-Derivat nach Anspruch 1, **dadurch gekennzeichnet, dass** die Substituenten (R⁴, R⁵, R⁶) an dem Silikon der Silyloxymethyl-Gruppe in 2'-O-Position zumindest teilweise miteinander verbunden sind.

5. Ribonukleosid-Derivat nach Anspruch 1, **dadurch gekennzeichnet, dass** die Substituenten (R⁴, R⁵, R⁶) an dem Silikon der Silyloxymethyl-Gruppe in 2'-O-Position Isopropyl-Gruppen sind.

6. Ribonukleosid-Derivat nach Anspruch 1, **dadurch gekennzeichnet, dass** die Base (R¹) Cytosin, Guanin, Adenin, Uracil oder Thymin ist.

7. Ribonukleosid-Derivat nach Anspruch 1, **dadurch gekennzeichnet, dass** die Base (R¹) eine Acyl-Schutzgruppe trägt.

8. Ribonukleosid-Derivat nach Anspruch 1, **dadurch gekennzeichnet, dass** das substituierte Derivat der phosphonischen Säure (R²) ein O- und N,N-substituierte Amino-Phosphino-Gruppe ist.

9. Ribonukleosid-Derivat nach Anspruch 8, **dadurch gekennzeichnet, dass** der O-Substituent eine 2-Cyanoethyl-Gruppe und dass die N-Substituenten Isopropyl-Gruppen sind.

10. Ribonukleosid-Derivat nach Anspruch 1, **dadurch gekennzeichnet, dass** die Schutzgruppe (R³) des Sauerstoffs in der 5'-Position eine Monomethoxytrityl- oder eine Dimethoxytrityl-Gruppe ist.

11. Verwendung des Ribonukleosid-Derivates nach Anspruch 1 für die chemische Synthese von RNA-Oligonukleotiden mit einer vorbestimmten Nukleotid-Sequenz.

12. Verfahren zum Herstellen des Ribonukleosid-Derivates nach Anspruch 1, **dadurch gekennzeichnet, dass** ein zyklisches 2', 3'-Di-O-Stannyl-Derivat in der Gegenwart einer Base aus einem Ribonukleosid hergestellt wird, wobei der Sauerstoff in der 5'-Position durch eine Schutzgruppe geschützt wird, und dass dieses Stannyl-Derivat durch das Hinzufügen eines Silyloxymethylhalogenids, welches drei zusätzliche Substituenten an dem Siliziumatom trägt, in das Ribonukleosid-Derivat konvertiert wird.

13. Verfahren nach Anspruch 12, **dadurch gekennzeichnet, dass** in einem weiteren Schritt das Ribonukleosid-Derivat an dem Sauerstoff in der 3'-Position mit einer Gruppe, die ein Derivat einer phosphonischen Säure umfasst, substituiert wird.

14. Ein Silyloxymethylhalogenid der Formel 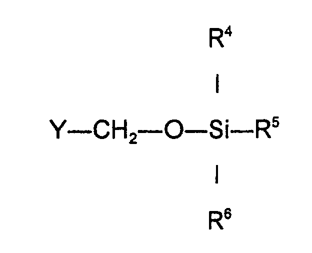 wobei Y ein Halogen und R⁴, R⁵ und R⁶ Isopropyle sind.

15. Ein Silyloxymethylhalogenid der Formel 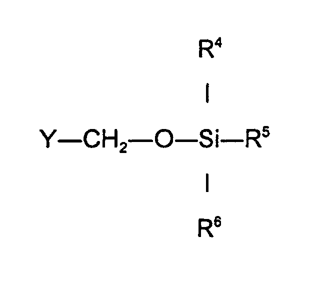 wobei Y ein Halogen und R⁴, R⁵ und R⁶ Alkyl- oder Arylsubstituenten sind, welche zusammen zwischen sechs und dreissig C-Atomen umfassen und zumindest teilweise untereinander verbunden sind.

## Revendications

1. Dérivé de nucléoside comprenant un motif D- ou L-ribose et répondant à la formule structurelle suivante : 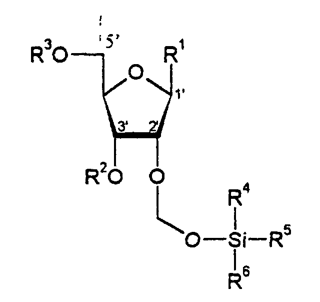 dans laquelle
R¹ est une base de la famille de la purine ou de la pyrimidine ou un dérivé d'une telle base,
R² est un proton ou un dérivé substitué d'acide phosphonique,
R³ est un proton ou un groupe de protection convenable,
et R⁴, R⁵ et R⁶ sont trois substituants aryle ou alkyle comprenant ensemble de six à trente atomes de carbone.

2. Dérivé de ribonucléoside selon la revendication 1, **caractérisé en ce que** les substituants (R⁴, R⁵, R⁶) sur le silicium du groupe silyloxyméthyle en position 2'-O sont au moins partiellement des groupes alkyle à substitution aryle ou des groupes aryle à substitution alkyle.

3. Dérivé de ribonucléoside selon la revendication 1, **caractérisé en ce que** les substituants (R⁴, R⁵, R⁶) sur le silicium du groupe silyioxyméthyle en position 2'-O sont au moins partiellement substitués par des hétéroatomes.

4. Dérivé de ribonucléoside selon la revendication 1, **caractérisé en ce que** les substituants (R⁴, R⁵, R⁶) sur le silicium du groupe silyloxyméthyle en position 2'-O sont au moins partiellement interconnectés.

5. Dérivé de ribonucléoside selon la revendication 1, **caractérisé en ce que** les substituants (R⁴, R⁵, R⁶) sur le silicium du groupe silyloxyméthyle en position 2'-O sont des groupes isopropyle.

6. Dérivé de ribonucléoside selon la revendication 1, **caractérisé en ce que** la base (R¹) est la cytosine, la guanine, l'adénine, l'uracile ou la thymine.

7. Dérivé de ribonucléoside selon la revendication 1, **caractérisé en ce que** la base (R¹) porte un groupe de protection acyle.

8. Dérivé de ribonucléoside selon la revendication 1, **caractérisé en ce que** le dérivé substitué d'acide phosphonique (R²) est un groupe aminophosphino O- et N,N-substitué.

9. Dérivé de ribonucléoside selon la revendication 8, **caractérisé en ce que** le substituant sur O est un groupe 2-cyanoéthyle et les substituants sur N sont des groupes isopropyle.

10. Dérivé de ribonucléoside selon la revendication 1, **caractérisé en ce que** le groupe de protection (R³) de l'oxygène en position 5' est un groupe monométhoxytrityle ou un groupe diméthoxytrityle.

11. Utilisation du dérivé de ribonucléoside selon la revendication 1, pour la synthèse chimique d'oligonucléotides d'ARN ayant une séquence de nucléotides prédéterminée.

12. Procédé pour la préparation du dérivé de ribonucléoside selon la revendication 1, **caractérisé en ce qu'**un dérivé de 2',3'-di-O-stannyle cyclique est préparé en présence d'une base à partir d'un ribonucléoside dont l'oxygène en position 5' est protégé par un groupe de protection et **en ce que** ce dérivé de stannyle est converti en le dérivé de ribonucléoside par addition d'un halogénure de silyloxyméthyle portant trois substituants additionnels sur l'atome de silicium.

13. Procédé selon la revendication 12, **caractérisé en ce que**, dans une étape ultérieure, le dérivé de ribonucléoside est substitué sur l'oxygène en position 3' par un groupe comprenant un dérivé d'acide phosphonique.

14. Halogénure de silyloxyméthyle de formule : 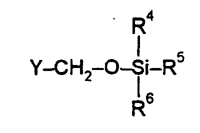 dans laquelle Y est un halogène et R⁴, R⁵ et R⁶ sont des radicaux isopropyle.

15. Halogénure de silyloxyméthyle de formule : 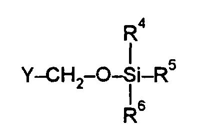 dans laquelle Y est un halogène et R⁴, R⁵ et R⁶ sont des substituants alkyle ou aryle comprenant ensemble entre six et trente atomes de carbone et sont au moins partiellement interconnectés.
